# EUROPEAN PATENT SPECIFICATION

(11) **EP 2 566 443 B1**
(45) Date of publication and mention of the grant of the patent: **02.11.2016**
(21) Application number: 10723803.2
(22) Date of filing: 07.05.2010
(51) Int. Cl.: A61K 8/44, A61Q 5/10, A61K 8/41, A61K 8/81

(54) **READY-TO-USE COSMETIC COMPOSITION FOR OXIDATION DYEING KERATIN FIBERS**
GEBRAUCHSFERTIGE KOSMETISCHE ZUSAMMENSETZUNG ZUR OXIDATIVEN FÄRBUNG VON KERATINFASERN
COMPOSITION COSMÉTIQUE PRÊT À L'USAGE POUR LA COLORATION PAR OXYDATION DE FIBRES DE KÉRATINE

(43) Date of publication of application: 13.03.2013
(73) Proprietor: L'Oréal, 75008 Paris (FR)
(72) Inventor: MISU Daisuke, Kawasaki-shi, Kanagawa 213-0012 (JP); PATAUT Francoise, Kawasaki-shi, Kanagawa 213-0012 (JP)
(74) Representative: Dossmann, Gérard
(86) International application number: PCT/JP2010/058146
(87) International publication number: WO 2011/138838

(56) References cited:
- EP-A1- 1 905 421
- WO-A1-02/087526
- WO-A1-2011/003553
- WO-A2-02/100363
- DE-A1- 4 120 361
- US-A- 5 785 961
- US-A1- 2002 157 191
- US-B1- 6 383 232
- "Chapter 31: Hair Colorants" In: Ralph Gordon Harry: "Harry's Cosmeticology 8th Edition", 1 April 2000 (2000-04-01), Chemical Publishing Company, New York, XP002618597, ISBN: 0820603724 pages 687-690, page 688, line 30 - page 690, line 13

## Description

### TECHNICAL FIELD

The present invention relates to a ready-to-use cosmetic composition for oxidation dyeing keratin fibers such as hair.

### BACKGROUND ART

It is known to dye keratin fibers, in particular hair, with dyeing compositions containing oxidation dyes. Oxidation dyes typically comprise oxidation dye precursors and couplers. Oxidation dye precursors, generally called oxidation bases, are colorless or weakly colored compounds which, combined with oxidizing agents, can give rise to colored and dye compounds by a process of oxidative condensation. They are in particular ortho- or para-phenylenediamines, ortho- or para-aminophenols, or heterocyclic bases. The shades obtained with these oxidation bases may be modified by combining the oxidation bases with couplers or color modifiers, the couplers being chosen in particular from aromatic meta-diamines, meta-aminophenols, meta-diphenols and certain heterocyclic compounds. The variety of molecules used in oxidation bases and couplers allows a rich palette of colors to be obtained.

The oxidation dyeing method generally consists in applying to keratin fibers oxidation bases or a mixture of oxidation bases and couplers with an oxidizing agent, most often hydrogen peroxide, in allowing to act, and then in rinsing the keratin fibers.

Due to the reactivity between the oxidation bases with or without couplers and the oxidizing agent, they generally have to be separately formulated into two parts - (1) a coloration part which contains the oxidation bases with or without couplers, and (2) a developer part which contains the oxidizing agent. Both of the two parts are mixed just before coloring the keratin fibers, and applied to the keratin fibers.

Such two-part hair dyeing compositions are in particular described in EP 1 905 421, DE41 20 361, US 6 383 232 and US 5 785 961. EP 1 905 421 describes a hair dye composition which is provided by mixing at least a first-part agent containing an alkaline agent and a second-part agent containing an oxidizing agent upon application, wherein the pH after mixing is from 8 to 12. It comprises, after mixing, the following components (a) to (c) and optionally an oxidative dye (d): (a) a betaine compound represented by formula (1) and an acid-addition salt thereof: R1R2R3N+-X-Y (1) wherein R1, R2 and R3 are the same or different from one another and each represents a C1-3 alkyl group, X represents a C1-3 alkylene group, and Y represents CO2-, SO3-, OSO3- or OPO3- in an amount ranging from 0.1 to 20 % by weight of the hair dye composition ; (b) an oxidizing agent; and (c) ammonia or a salt thereof. DE 41 20 361 describes a ready-to-use hair dyeing composition obtained by mixing two agents at the time of application. After mixing, it comprises at least one oxidation dye, at least one oxidizing agent and from 2.5 to 15 % by weight of C1-18 alkylamidopropylbetaine relative to the total weight of the composition. US 6 383 232 describes ready to use water-based formulations for oxidative hair dyeing containing oxidation dyes, 6 wt% of amidobetaine derived from meadow foam seed oil, hydrogen peroxide, a cationic polymer and a higher alcohol. Finally US 5 785 961 describes oxidative hair dye compositions in aqueous medium containing oxidation dyes, between 1.5 to 10 wt% of a mixture of amphoteric surfactants including undecyl-N-hydroxy-N-carboxymethylimidazolinium betaine, cocoyl amide propyldimethyl glycine and sodium lauroamphoacetate, and a cationic polymer.

Tn order to facilitate the coloring of keratin fibers, each of oxidation bases, couplers and oxidizing agents is solved or dispersed in a medium, usually in the form of a powder, cream, gel, and the like, in each of the coloration part and the developer part.

The media for coloring keratin fibers make it easy to handle coloring products for keratin fibers. However, additional production processes and costs are necessary to prepare the formulations for the coloration part and the developer part. In addition, it is necessary to carefully select the media to ensure the compatibility with active substances such as oxidation bases, couplers and oxidizing agents.

### DISCLOSURE OF INVENTION

An object of the present invention is to provide a cosmetic composition for oxidation dyeing keratin fibers such as hair, which can be prepared in a simple and inexpensive process, and can avoid the above potential compatibility issue.

Thus, one aspect of the present invention is a cosmetic composition for oxidation dyeing keratin fibers comprising, in an aqueous medium:
at least one oxidation dye;
at least one oxidizing agent; and
1.5 wt% or more of at least one amphoteric surfactant, relative to the total weight of the composition.

It is preferable that the composition according to the present invention comprises water in an amount of 50 wt% or more relative to the total weight of the composition.

It is preferable that the type of the amphoteric surfactant be a betaine.

The betaine-type amphoteric surfactant may be selected from the group consisting of alkylbetaines, sulfobetaines, alkylamidoalkylbetaines and alkylamidoalkylsulfobetaines.

The betaine-type amphoteric surfactant may preferably be an alkylbetaine.

The oxidation dye may be selected from oxidation bases, oxidation couplers, and the acid addition salts thereof.

The oxidation dye may be an oxidation base selected from the group consisting of ortho- and para-phenylenediamines, double bases, ortho- and para-aminophenols, heterocyclic bases and the acid addition salts thereof.

The oxidation dye may be an oxidation coupler selected from the group consisting of meta-phenylenediamines, meta-aminophenols, meta-diphenols, naphthols, heterocyclic couplers and the acid addition salts thereof.

The amount of the oxidation dye(s) may be 0.0001 to 20 wt%, preferably 0.0005 to 15 wt%, and more preferably 0.005 to 10 wt%, relative to the total weight of the composition.

The oxidizing agent may be selected from the group consisting of hydrogen peroxide, urea peroxide, alkali metal bromates, persalts, peracids and oxidase enzymes. Preferably, the oxidizing agent is hydrogen peroxide.

The amount of the oxidizing agent(s) may be 0.0001 to 10-wt%, preferably 0.001 to 10 wt%, and more preferably 0.01 to 5 wt%, relative to the total weight of the composition.

The amount of the amphoteric surfactant(s) may be 1.5 to 10 wt%, preferably 1.8 to 5 wt%, and more preferably 1.8 to 3 wt%, relative to the total weight of the composition.

The composition according to the present invention may further comprise at least one cationic polymer. The amount of the cationic polymer may be 0.0001 to 10 wt%, preferably 0.001 to 10 wt%, and more preferably 0.01 to 5 wt%, relative to the total weight of the composition.

The composition according to the present invention may further comprise at least one higher alcohol. The amount of the higher alcohol may be 0.0001 to 10 wt%, preferably 0.001 to 10 wt%, and more preferably 0.01 to 5 wt%, relative to the total weight of the composition.

The composition according to the present invention may be obtained prior to use by mixing a first composition comprising one or several oxidation dyes, and a second composition comprising one or several oxidizing agents wherein one or several amphoteric surfactants is/are present in either or both of the first and second compositions, the concentration of amphoteric surfactant(s) in the mixture being 1.5 wt% or more relative to the total weight of the compositions.

### BRIEF DESCRIPTION OF DRAWINGS

Figure 1 shows a graph of friction coefficients of Example 1 and Comparative Example 1.
Figure 2 shows a graph of color intensity of Example 1 and Comparative Example 2.

### BEST MODE FOR CARRYING OUT THE INVENTION

The inventors performed diligent research and found that an aqueous or water-based formulation containing a relatively large amount of at least one amphoteric surfactant can solve the problems of complicated preparation process and additional costs, which are found in conventional cosmetic products for oxidation dyeing keratin fibers.

Since the aqueous formulation does not require a large amount of fatty substance(s) such as oil(s), which is/are normally used with a large amount in the media for conventional cosmetic products, in particular cream type media, the procedure for emulsifying the fatty substance(s) is unnecessary, and therefore, the entire process for preparing the formulation can be simplified and cost effective.

In addition, since active substances such as oxidation bases, couplers and oxidizing agents are easily solved in water, the issue of compatibility with media can be minimized.

It was also found by the inventors that better cosmetic properties such as a smooth feeling on touch and better color intensity can be provided by using at least one amphoteric surfactant, preferably in relatively a large amount. This may be attributed to better penetration of oxidation dyes and/or oxidizing agents to keratin fibers.

Furthermore, since the amphoteric surfactant(s) is/are present in a relatively large amount in the cosmetic composition according to the present invention, the cosmetic composition can be foamed by hand or by using an appropriate apparatus or equipment. For example, the cosmetic composition according to the present invention can be dispensed in the form of a foaming composition from a dispenser (aerosol or pump-dispenser bottle device) such as an spray. The foaming composition is easy to handle because it is difficult to drip off from keratin fibers, and easy to stay on and in keratin fibers.

Thus, the aqueous cosmetic composition according to the present invention is characterized by comprising, in an aqueous medium:
(1) at least one oxidative dye;
(2) at least one oxidation agent; and
(3) 1.5 wt% or more of at least one amphoteric surfactant, relative to the total weight of the composition.

Hereinafter, the cosmetic composition according to the present invention will be explained in a more detailed manner.

### (1) Aqueous Medium

The aqueous medium in the cosmetic composition according to the present invention comprises water. The amount of water may be 50 wt% or more, preferably 60 wt% or more, more preferably 70 wt% or more, and further more preferably 80 wt% or more, relative to the total weight of the composition.

The aqueous medium may further comprise at least one organic solvent for solubilizing the compounds which might not be sufficiently soluble in water. Two or more of the organic solvent(s) may be used. The organic solvent is preferably water-miscible. As the organic solvent, there may be mentioned, for example, C₁-C₄ alkanols, such as ethanol and isopropanol; glycerol; glycols and glycol ethers such as 2-butoxyethanol, propylene glycol, monomethyl ether of propylene glycol, monoethyl ether and monomethyl ether of diethylene glycol, and aromatic alcohols such as benzyl alcohol and phenoxyethanol, analogous products and mixtures thereof.

The organic solvent may be present in an amount ranging from 1 to 40 wt%, preferably from 1 to 30 wt%, and more preferably from 5 to 20 wt%, relative to the total weight of the composition.

### (2) Oxidation Dye

The cosmetic composition according to the present invention comprises at least one oxidation dye. Two or more of the oxidative dye(s) may be used.

The oxidation dye can be selected from oxidation bases, oxidation couplers, and the acid addition salts thereof.

The oxidation base can be selected from those conventionally known in oxidation dyeing, preferably from the group consisting of ortho- and para-phenylenediamines, double bases, ortho- and para-aminophenols, heterocyclic bases and the acid addition salts thereof.

There may be mentioned in particular:
- (I) the para-phenylenediamines of the following formula (I) and their addition salts with an acid: 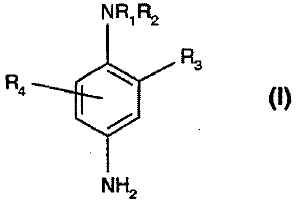 in which:
   R₁ represents a hydrogen atom, a C₁-C₄ alkyl radical, a monohydroxy(C₁-C₄ alkyl) radical, a polyhydroxy-(C₂-C₄ alkyl) radical, a (C₁-C₄) alkoxy (C₁-C₄) alkyl radical, a C₁-C₄ alkylradical substituted with a nitrogen-containing group, a phenyl radical or a 4'-aminophenyl radical;
   R₂ represents a hydrogen atom, a C₁-C₄ alkyl radical, a monohydroxy(C₁-C₄ alkyl) radical, a polyhydroxy(C₂-C₄ alkyl) radical, a (C₁-C₄) alkoxy (C₁-C₄) alkyl radical or a C₁-C₄ alkyl radical substituted with a nitrogen-containing group;
   R₁ and R₂ may also form with the nitrogen atom carrying them a 5- or 6-membered nitrogen-containing heterocycle optionally substituted with one or more alkyl, hydroxyl or ureido groups;
   R₃ represents a hydrogen atom, a halogen atom such as a chlorine atom, a C₁-C₄ alkyl radical, a sulpho radical, a carboxyl radical, a monohydroxy(C₁-C₄ alkyl) radical, a hydroxy(C₁-C₄ alkoxy) radical, an acetylamino(C₁-C₄ alkoxy) radical, a mesylamino(C₁-C₄ alkoxy) radical or a carbamoylamino(C₁-C₄ alkoxy) radical; and R₄ represents a hydrogen or halogen atom or a C₁-C₄ alkyl radical.

   Among the nitrogen-containing groups of formula (I) above, there may be mentioned in particular the amino, mono(C₁-C₄)alkylamino, (C₁-C₄) dialkylamino, (C₁-C₄) trialkylamino, monohydroxy (C₁-C₄)alkylamino, di(monohydroxy(C₁-C₄)alkyl)amino, imidazolinium and ammonium radicals.
   Among the para-phenylenediamines of formula (I) above, there may be mentioned more particularly para-phenylenediamine, paratolylenediamine, 2-chloro-paraphenylenediamine, 2,3-dimethyl-paraphenylenediamine, 2,6-dimethyl-para-phenylenediamine, 2,6-diethyl-para-phenylenediamine, 2,5-dimethyl-para-phenylenediamine, N,N-dimethylpara-phenylenediamine, N,N-diethyl-para-phenylenediamine, N,N-dipropyl-paraphenylenediamine, 4-amino-N,N-diethyl-3-methylaniline, N,N-bis(β-hydroxyethyl)-paraphenylenediamine, 4-N,N-bis(β-hydroxyethyl)amino-2-methylaniline, 4-N,N-bis(β-hydroxyethyl)amino-2-chloroaniline, 2-β-hydroxyethyl-paraphenylenediamine, 2-fluoro-paraphenylenediamine, 2-isopropyl-paraphenylenediamine, N-(β-hydroxypropyl)-paraphenylenediamine, 2-hydroxymethyl-para-phenylenediamine, N,N-dimethyl-3-methylparaphenylenediamine, N,N-(ethyl-β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylamino-ethyloxy-para-phenylenediamine, N-(β-methoxyethyl)-para-phenylenediamine, 2-methyl-1-N-β-hydroxyethyl-para-phenylenediamine, N-(4-aminophenyl)-3-hydroxy-pyrrolidine, 2-[{2-[(4-Aminophenyl)amino]ethyl}(2-hydroxyethyl)amino]-ethanol, and their addition salts with an acid.
   Among the para-phenylenediamines of formula (I) above, there are most particularly preferred para-phenylenediamine, paratolylenediamine, 2-isopropyl-paraphenylenediamine, 2-β-hydroxyethyl-para-phenylenediamine, 2-β-hydroxyethyloxy-paraphenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-paraphenylenediamine, and their addition salts with an acid.
- (II) According to the invention, "double bases" is understood to mean compounds containing at least two aromatic rings on which amino and/or hydroxyl groups are carried.
   Among the double bases which can be used as oxidation bases in the dyeing compositions in accordance with the invention, there may be mentioned in particular compounds corresponding to the following formula (II), and their addition salts with an acid: 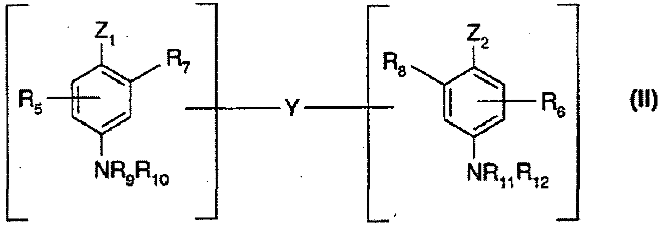 in which:
   - Z₁ and Z₂, which are identical or different, represent a hydroxyl or -NH₂ radical which may be substituted with a C₁-C₄ alkyl radical or with a linking arm Y;
   - the linking arm Y represents a linear or branched alkylene chain comprising from 1 to 14 carbon atoms, which may be interrupted by or which may end with one or more nitrogen-containing groups and/or one or more heteroatoms such as oxygen, sulphur or nitrogen atoms, and optionally substituted with one or more hydroxyl or C₁-C₆ alkoxy radicals;
   - R₅ and R₆ represent a hydrogen or halogen atom, a C₁-C₄ alkyl radical, a monohydroxy(C₁-C₄ alkyl) radical, a polyhydroxy(C₂-C₄ alkyl) radical, an amino(C₁-C₄ alkyl) radical or a linking arm Y;
   - R₇, R₈, R₉, R₁₀, R₁₁ and R₁₂, which are identical or different, represent a hydrogen atom, a linking arm Y or a C₁-C₄ alkyl radical; it being understood that the compounds of formula (II) contain only one linking arm Y per molecule.

   Among the nitrogen-containing groups of formula (II) above, there may be mentioned in particular the amino, mono(C₁-C₄)alkylamino, (C₁-C₄) dialkylamino, (C₁-C₄) trialkylamino, monohydroxy(C₁-C₄)alkylamino, imidazolinium and ammonium radicals.
   Among the double bases of formulae (II) above, there may be mentioned more particularly N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)-tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylene-diamine, 1,8-bis(2,5-diaminophenoxy)-3,5-dioxaoctane, and their addition salts with an acid.
   Among these double bases of formula (II), N,N'-bis(β-hydroxyethyl)-N,N'-bis(4'-aminophenyl)-1,3-diaminopropanol, 1,8-bis(2,5-diaminophenoxy)-3,5-dioxaoctane or one of their addition salts with an acid are particularly preferred.
- (III) The para-aminophenols corresponding to the following formula (III), and their addition salts with an acid: 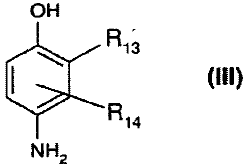 in which:
   - R₁₃ represents a hydrogen atom, or a halogen atom such as fluorine, a C₁-C₄ alkyl, monohydroxy (C₁-C₄alkyl), (C₁-C₄)alkoxy(C₁-C₄)-alkyl, amino (C₁-C₄ alkyl) or hydroxy (C₁-C₄) alkylamino- (C₁-C₄ alkyl) radical,
   - R₁₄ represents a hydrogen atom, or a halogen atom such as fluorine, a C₁-C₄ alkyl, monohydroxy (C₁-C₄ alkyl), polyhydroxy(C₂-C₄alkyl), amino (C₁-C₄ alkyl), cyano (C₁-C₄ alkyl) or (C₁-C₄) alkoxy (C₁-C₄) alkyl radical.

   Among the para-aminophenols of formula (III) above, there may be mentioned more particularly para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, and their addition salts with an acid.
- (IV) The ortho-aminophenols which can be used as oxidation bases in the context of the present invention are chosen in particular from 2-aminophenol, 2-amino-1-hydroxy-5-methylbenzene, 2-amino-1-hydroxy-6-methylbenzene, 5-acetamido-2-aminophenol, and their addition salts with acid.
- (V) Among the heterocyclic bases which can be used as oxidation bases in the dyeing compositions in accordance with the invention, there may be mentioned more particularly pyridine derivatives, pyrimidine derivatives, pyrazole derivatives, and their addition salts with an acid.

Among the pyridine derivatives, there may be mentioned more particularly the compounds described for example in Patents GB 1,026,978 and GB 1,153,196, such as 2,5-diaminopyridine, 2-(4-methoxyphenyl)amino-3-aminopyridine, 2,3-diamino-6-methoxypyridine, 2-(β-methoxyethyl)amino-3-amino-6-methoxypyridine, 3,4-diaminopyridine, and their addition salts with an acid.

Among the pyrimidine derivatives, there may be mentioned more particularly the compounds described, for example, in Patents DE 2 359 399; JP 88-169571; JP 91-10659 or patent application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triamino-pyrimidine, and the pyrazolopyrimidine derivatives such as those mentioned in patent application FR-A-2 750 048 and among which there may be mentioned pyrazolo[1,5-a]-pyrimidine-3,7-diamine; 2,5-dimethyl-pyrazolo[1,5-a]-pyrimidine-3,7-diamine; pyrazolo[1,5-a]pyrimidine-3,5-diamine; 2,7-dimethylpyrazolo[1,5-a]pyrimidine-3,5-diamine; 3-aminopyrazolo[1,5-a]pyrimidin-7-ol; 3-amino-pyrazolo[1,5-a]pyrimidin-5-ol; 2-(3-amino-pyrazolo-[1,5-a]pyrimidin-7-ylamino)ethanol, 2-(7-aminopyrazolo[1,5-a]pyrimidin-3-ylamino)ethanol, 2-[(3-amino-pyrazolo[1,5-a]pyrimidin-7-yl)-(2-hydroxy-ethyl)amino]-ethanol, 2-[(7-aminopyrazolo[1,5-a]-pyrimidin-3-yl)-(2-hydroxyethyl)amino]ethanol, 5,6-dimethylpyrazolo-[1,5-a]pyrimidine-3,7-diamine, 2,6-dimethylpyrazolo-[1,5-a]pyrimidine-3,7-diamine, 2,5,N7,N7-tetramethyl-pyrazolo[1,5-a]pyrimidine-3,7-diamine, 3-amino-5-methyl-7-imidazolylpropyl-aminopyrazolo[1,5-a]-pyrimidine, their addition salts and their tautomeric forms, when a tautomeric equilibrium exists and their addition salts with an acid.

Among the pyrazole derivatives, there may be mentioned more particularly the compounds described in Patents DE 3 843 892, DE 4 133 957 and patent applications WO 94/08969, WO 94/08970, FR-A-2 733 749 and DE 195 43 988 such as 4,5-diamino-1-methylpyrazole, 3,4-diaminopyrazole, 4,5-diamino-1-(4'-chlorobenzyl)-pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazino-pyrazole, 1-benzyl-4,5-diamino-3-methyl-pyrazole, 4,5-diamino-3-tert-butyl-1-methylpyrazole, 4,5-diamino-1-tertbutyl-3-methylpyrazole, 4,5-diamino-1-(β-hydroxyethyl)-3-methylpyrazole, 4,5-diamino-1-(β-hydroxyethyl)pyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxy-methylpyrazole, 4,5-diamino-3-hydroxymethyl-1-methylpyrazole, 4,5-diamino-3-hydroxymethyl-1-isopropyl-pyrazole, 4,5-diamino-3-methyl-1-isopropyl-pyrazole, 4-amino-5-(2'-aminoethyl)amino-1,3-dimethylpyrazole, 3,4,5-triaminopyrazole, 1-methyl-3,4,5-triamino-pyrazole, 3,5-diamino-1-methyl-4-methylaminopyrazole, 3,5-diamino-4-(β-hydroxy-ethyl)amino-1-methylpyrazole, and their addition salts with an acid.

Among the heterocyclic bases which can be used as oxidation bases, there may be mentioned more particularly diaminopyrazolopyrazolones and especially 2,3-diamino-6,7-dihydro-1H5H-[pyrazolo1,2,a]pyrazol-1-one and the addition salts of these diaminopyrazolopyrazolones with an acid.

The oxidation dye may be an oxidation coupler which can be selected from those conventionally known in oxidation dyeing, preferably from the group consisting of meta-phenylenediamines, meta-aminophenols, meta-diphenols, naphthols, heterocyclic couplers and the acid addition salts thereof.

The heterocyclic couplers may be selected from the group consisting of indole derivatives, indoline derivatives, sesamol and its derivatives, pyridine derivatives, pyrazolotriazole derivatives, pyrazolones, indazoles, benzimidazoles, benzothiazoles, benzoxazoles, 1,3-benzodioxoles, quinolines and their addition salts with an acid.

These couplers are more particularly chosen from 2,4-diamino-1-(β-hydroxyethyloxy)benzene, 2-methyl-5-aminophenol, 5-N-(β-hydroxyethyl)amino-2-methylphenol, 3-aminophenol, 2-chloro-3-amino-6-methylphenol, 1,3-dihydroxybenzene, 1,3-dihydroxy-2-methylbenzene, 4-chloro-1,3-dihydroxybenzene, 2-amino-4-(β-hydroxyethylamino)-1-methoxybenzene, 1,3-diaminobenzene, 2-methyl-5-hydroxyethylaminophenol, 4-amino-2-hydroxytoluene, 1,3-bis(2,4-diaminophenoxy)-propane, sesamol, 1-amino-2-methoxy-4,5-methylene-dioxybenzene, α-naphthol, 6-hydroxyindole, 4-hydroxyindole, 4-hydroxy-N-methylindole, 6-hydroxy-indoline, 2,6-dihydroxy-4-methylpyridine, 1-H-3-methylpyrazol-5-one, 1-phenyl-3-methylpyrazol-5-one, 2-amino-3-hydroxypyridine, 3,6-dimethyl-pyrazolo[3,2-c]-1,2,4-triazole, 2,6-dimethylpyrazolo[1,5-b]-1,2,4-triazole and their addition salts with an acid.

In general, the addition acid salts of the oxidation bases and couplers are chosen in particular from hydrochlorides, hydrobromides, sulphates, tartrates, lactates and acetates.

The cosmetic composition according to the present invention may comprise an oxidation dye or dyes in an amount of 0.0001 to 20 wt%, preferably 0.0005 to 15 wt%, and more preferably 0.005 to 10 wt%, relative to the total weight of the composition.

### (3) Oxidizing agent

The cosmetic composition according to the present invention comprises at least one oxidizing agent. Two or more of the oxidizing agents may be used.

The oxidizing agent may be chosen from hydrogen peroxide, peroxygenated salts, and compounds capable of producing hydrogen peroxide by hydrolysis. For example, the oxidizing agent can be chosen from aqueous hydrogen peroxide solution, urea peroxide, alkali metal bromates and ferricyanides and persalts such as perborates and persulphates. At least one oxidase enzyme chosen, for example, from laccases, peroxidases and 2-electron oxidoreductases such as uricase may also be used as the oxidizing agent, where appropriate in the presence of the respective donor or co-factor thereof.

In one embodiment, the oxidizing agent is hydrogen peroxide, such as an aqueous hydrogen peroxide solution.

The hydrogen peroxide concentration may range from 0.15 wt% to 12 wt% such as from 0.6 wt% to 9 wt% relative to the total weight of the oxidizing agent. The concentration of compounds capable of forming hydrogen peroxide by hydrolysis may range from 0.1 wt% to 25 wt% relative to the total weight of the oxidizing agent.

In one embodiment, when the oxidizing agent is an aqueous hydrogen peroxide solution, the composition may comprise at least one hydrogen peroxide stabilizer, which may be chosen, for example, from alkali metal and alkaline-earth metal pyrophosphates, alkali metal and alkaline-earth metal stannates, phenacetin and salts of acids and of oxyquinoline, for example, oxyquinoline sulphate. In another embodiment, at least one stannate optionally in combination with at least one pyrophosphate is used.

It is also possible to use salicylic acid and its salts, pyridinedicarboxylic acid and its salts, paracetamol.

In the oxidizing agent, the concentration of the hydrogen peroxide stabilizer may range from 0.0001 wt% to 5 wt% such as from 0.01 wt% to 2 wt% relative to the total weight of the oxidizing agent.

In the composition comprising an aqueous hydrogen peroxide solution, the concentration ratio of the hydrogen peroxide to the at least one stabilizer may range from 0.05:1 to 1,000:1, such as from 0.1:1 to 500:1 and further such as from 1:1 to 200:1.

The amount of the oxidizing agent(s) may range from 0.0001 to 10 wt%, preferably 0.001 to 10 wt%, and more preferably 0.01 to 5 wt%, relative to the total weight of the composition.

### (4) Amphoteric Surfactant

The cosmetic composition according to the present invention comprises at least one amphoteric surfactant. Two or more of the amphoteric surfactants may be used.

The amphoteric surfactant is not limited. The amphoteric or zwitterionic surfactants can be, for example (nonlimiting list), aliphatic secondary or tertiary amine, and optionally quaternized amine derivatives, in which the aliphatic radical is a linear or branched chain comprising 8 to 22 carbon atoms and containing at least one water-solubilizing anionic group (for example, carboxylate, sulphonate, sulphate, phosphate or phosphonate).

Among the amine derivatives, mention may be made of the products sold under the name Miranol, as described in U.S. Pat. Nos. 2,528,378 and 2,781,354 and classified in the CTFA dictionary, 3rd edition, 1982 (the disclosures of which are incorporated herein by reference), under the names Amphocarboxyglycinates and Amphocarboxypropionates, with the respective structures:

R₃₄-CONHCH₂CH₂-N⁺(R₃₅) (R₃₆) (CH₂COO⁻)

in which:
R₃₄ denotes an alkyl radical of an acid R₃₄-COOH present in hydrolysed coconut oil, a heptyl, nonyl or undecyl radical,
R₃₅ denotes a beta-hydroxyethyl group, and
R₃₆ denotes a carboxymethyl group; and

R₃₄'-CONHCH₂CH₂-N(B) (C)

in which:
B represents -CH₂CH₂OX',
C represents -(CH₂)_{z}-Y', with z=1 or 2,
X' denotes a -CH₂CH₂-COOH group, -CH₂-COOZ', -CH₂CH₂-COOH, -CH₂CH₂-COOZ' or a hydrogen atom,
Y' denotes -COOH, -COOZ', -CH₂-CHOH-SO₃Z' or a -CH₂-CHOH-SO₃H radical,
Z' represents ion of alkaline or alkaline earth metal such as sodium, ammonium ion or ion issued from an organic amine, and
R₃₄' denotes an alkyl radical of an acid R₃₇-COOH present in coconut oil or in hydrolysed linseed oil, an alkyl radical, such as a C₇, C₉, C₁₁ or C₁₃ alkyl radical, a C₁₇ alkyl radical and its iso form, or an unsaturated C₁₇ radical.

These compounds are classified in the CTFA dictionary, 5th edition, 1993, under the names Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphopropionate, Disodium Caprylamphodipropionate, Disodium Caprylamphodipropionate, Lauroamphodipropionic acid and Cocoamphodipropionic acid.

By way of example, mention may be made of the cocoamphodiacetate sold under the trade name Miranol® C2M concentrate by the company Rhodia Chimie.

Preferably, the amphoteric surfactant may be a betaine.

The betaine-type amphoteric surfactant is preferably selected from the group consisting of alkylbetaines, sulfobetaines, alkylamidoalkylbetaines and alkylamidoalkylsulfobetaines, in particular, (C₈-C₂₀)alkylbetaines, sulphobetaines, (C₈-C₂₀)alkyla mido(C₁-C₈)alkylbetaines and (C₈-C₂₀)alkylamido(C₁-C₈)alkylsulphobetaines. In one embodiment, the amphoteric surfactants of betaine type are chosen from (C₈-C₂₀)alkylbetaines, (C₈-C₂₀)alkylamido(C₁-C₈)alkylsulfobetaines, and sulfobetaines.

Non-limiting examples that may be mentioned include the compounds classified in the CTFA dictionary, 9th edition, 2002, under the names cocobetaine, laurylbetaine, cetylbetaine, coco/oleamidopropylbetaine, cocamidopropylbetaine, palmitamidopropylbetaine, stearamidopropylbetaine, cocamidoethylbetaine, cocamidopropylhydroxysultaine, oleamidopropylhydroxysultaine, cocohydroxysultaine, laurylhydroxysultaine, and cocosultaine, alone or as mixtures.

The betaine-type amphoteric surfactant is preferably an alkylbetaine, in particular cocobetaine.

The amount of the amphoteric surfactant(s) must be 1.5wt% or more, and may range from 1.5 wt% to 10 wt%, preferably 1.8 wt% to 5 wt%, and more preferably 1.8 wt% to 3 wt%, relative to the total weight of the composition.

Generally, a large amount of amphoteric surfactant(s) is not used in cosmetic compositions because it typically causes the reduction of the viscosity of the cosmetic compositions, which may impair the usability of the cosmetic compositions. On the other hand, the cosmetic composition according to the present invention can comprise relatively a large amount of amphoteric surfactant(s), because it comprises an aqueous medium, and therefore it is aqueous or water-based.

### (5) Other Components

The cosmetic composition according to the present invention may further comprise at least one additional surfactant selected from nonionic, anionic and cationic surfactants. Two or more of the additional surfactants may be used.

Preferably, the composition according to the present invention does not contain anionic surfactants, or contains anionic surfactants in a weight ratio of amphoteric surfactants/anionic surfactants being higher than 1.

A specific embodiment of the composition according to the present invention contains one or several non-ionic surfactants.

The non-ionic surfactants can be selected from surfactants such as those described in "Handbook of Surfactants" par M.R. PORTER, editions Blackie & Son (Glasgow and London), 1991, pp 116-178.

They particularly can be selected from:
- alcohols, alpha-diols, alkylphenols which are polyethoxylated, polypropoxylated or polyglycerolated, and having a fatty chain comprising 8 to 30 carbon atoms, the number of ethylene oxide or propylene oxide groups ranging from 2 to 150 and the number of glycerol groups ranging from 2 to 30;
- copolymers of ethylene and propylene oxides;
- polyoxyethylenated fatty amides with 2 to 30 ethylene oxide groups;
- polyoxyglycerolated fatty amides with 1 to 5 glycerol groups;
- polyethoxylated vegetal oils with 2 to 150 ethylene oxide groups;
- polyoxyethylenated fatty esters of sorbitan with 2 to 30 ethylene oxide groups;
- fatty esters of sucrose;
- fatty esters of polyethylene glycols; and
- alkylpolyglycosides.

In another specific embodiment the composition of the invention contains only amphoteric surfactants as surfactants.

The cosmetic composition according to the present invention may further comprise at least one cationic polymer. Two or more of the cationic polymers may be used.

The term "cationic polymer" here is defined as any polymer comprising cationic groups and/or groups which may be ionized into cationic groups.

Representative cationic polymers which may be used in accordance with the present invention may be chosen from any of those already known in the art for improving the cosmetic properties of the hair, such as those described, for example, in patent application EP-A-337 354 and in French patents FR-2 270 846, 2 383 660, 2 598 611, 2 470 596 and 2 519 863 (the disclosures of which are incorporated herein by reference).

Cationic polymers which may be used include those cationic polymer comprising units which are comprised of primary, secondary, tertiary, and/or quaternary amine groups, which may either form part of the main polymer chain or may be borne by a side substituent directly attached thereto.

Said cationic polymers can have an approximate number-average molecular mass of from about 500 to about 5×10⁶ such as from about 10³ to about 3×10⁶.

Additional cationic polymers which may be mentioned are polymers of the polyamine, polyamino amide, and polyquaternary ammonium type.

Said cationic polymers are products that are well known to one of ordinary skill in the art. They are described, for example, in French patents 2 505 348 and 2 542 997 (the disclosures of which are incorporated herein by reference). Among said polymers, mention may be made, for instance, of:
(1) homopolymers or copolymers derived from acrylic esters, methacrylic esters, or amides and comprising at least one of the units of formula (V), (VI), (VII), or (VIII) below: 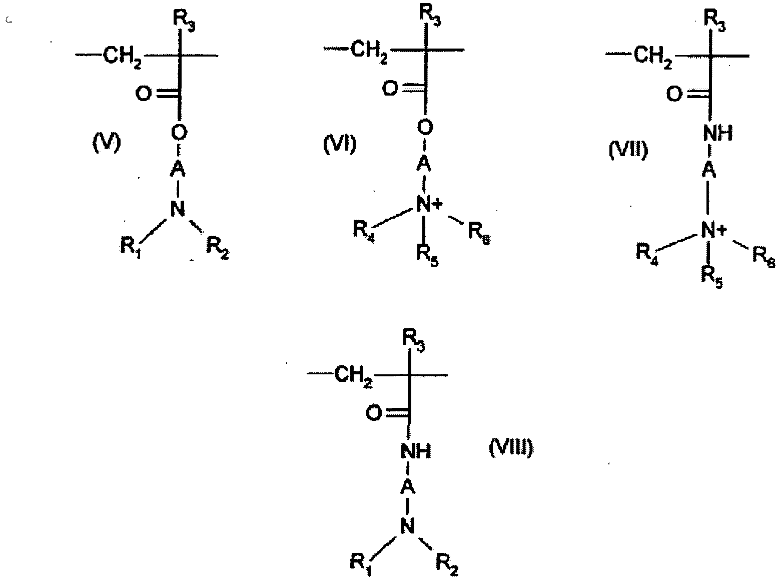 wherein
   R₃, which may be identical or different, denote a hydrogen atom or a CH₃ radical;
   A, which may be identical or different, represent a linear or branched alkyl group of 1 to 6 carbon atoms such as 2 or 3 carbon atoms, or a hydroxyalkyl group of 1 to 4 carbon atoms;
   R₄, R₅ and R₆, which may be identical or different, represent an alkyl group comprising 1 to 18 carbon atoms such as an alkyl group comprising from 1 to 6 carbon atoms, or a benzyl radical;
   R₁ and R₂, which may be identical or different, represent hydrogen or an alkyl group comprising from 1 to 6 carbon atoms, such as methyl or ethyl;
   X denotes an anion derived from an inorganic or organic acid, such as a methosulphate anion or a halide such as chloride or bromide.

   The homopolymers or copolymers of (1) can further comprise one or more units derived from comonomers which may be chosen from acrylamides, methacrylamides, diacetoneacrylamides, acrylamides, methacrylamides substituted on the nitrogen with lower (C₁-C₄) alkyls, acrylic, or methacrylic acids or esters thereof, vinyllactams such as vinylpyrrolidone or vinylcaprolactam, and vinyl esters.
   Thus, among homopolymers or copolymers of (1), mention may be made, for example, of:
   - copolymers of acrylamide and of dimethylaminoethyl methacrylate quaternized with dimethyl sulphate or with a dimethyl halide, such as the product sold under the name Hercofloc by the company Hercules,
   - the copolymers of acrylamide and of methacryloyloxyethyltrimethylammonium chloride described, for example, in patent application EP-A-080 976 and sold under the name Bina Quat P 100 by the company Ciba Geigy,
   - the copolymer of acrylamide and of methacryloyloxyethyltrimethylammonium methosulphate sold under the name Reten by the company Hercules,
   - quaternized or non-quaternized vinylpyrrolidone/dialkylaminoalkyl acrylate or methacrylate copolymers, such as the products sold under the name "Gafquat" by the company ISP, such as, for example, "Gafquat 734" or "Gafquat 755", or alternatively the products known as "Copolymer 845, 958 and 937", these polymers being described in detail in French patents 2 077 143 and 2 393 573,
   - dimethylaminoethyl methacrylate/vinylcaprolactam/vinylpyrrolidone terpolymers, such as the product sold under the name Gaffix VC 713 by the company ISP,
   - vinylpyrrolidone/methacrylamidopropyldimethylamine copolymers sold in particular under the name Styleze CC 10 by ISP, quaternized vinylpyrrolidone/dimethylaminopropylmethacrylamide copolymers such as the product sold under the name "Gafquat HS 100" by the company ISP,
   - crosslinked methacryloyloxy (C₁-C₄) alkyltri (C₁-C₄) alkylammonium salt polymers such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with a compound comprising olefinic unsaturation, such as methylenebisacrylamide,
   - a crosslinked acrylamide/methacryloyloxyethyltrimethylammonium chloride copolymer (20/80 by weight) in the form of a dispersion containing 50% by weight of the said copolymer in mineral oil can also be used, for example. This dispersion is sold under the name "Salcare® SC 92" by the company Allied Colloids, and
   - a crosslinked methacryloyloxyethyltrimethylammonium chloride homopolymer comprising about 50% by weight of the homopolymer in mineral oil or in a liquid ester can also be used. These dispersions, for example, are sold under the names "Salcare® SC 95" and "Salcare® SC 96" by the company Allied Colloids.
(2) Cationic cellulose derivatives such as:
   cellulose ether derivatives containing quaternary ammonium groups,
   described in French patent 1 492 597 (the disclosure of which is incorporated herein by reference), such as polymers sold under the names "JR" (JR 400, JR 125 and JR 30M) or "LR" (LR 400, or LR 30M) by the company Union Carbide Corporation. These polymers are also defined in the CTFA dictionary as quaternary ammoniums of hydroxyethylcellulose which has reacted with an epoxide substituted with a trimethylammonium group, and
   cellulose copolymers or cellulose derivatives grafted with a water-soluble monomer of quaternary ammonium, and described for example in U.S. Pat. No. 4,131,576 (the disclosure of which is incorporated herein by reference), such as hydroxyalkylcelluloses, for instance hydroxymethyl-, hydroxyethyl-, or hydroxypropylcelluloses grafted with a methacryloyloxyethyltrimethylammonium, methacrylamidopropyltrimethylammonium, or dimethyldiallylammonium salt, for instance.

   The commercial products corresponding to this definition, for instance, are sold under the names "Celquat L 200" and "Celquat H 100" by the company National Starch.
(3) The non-cellulosic cationic polysaccharides described, for example, in U.S. Pat. Nos. 3,589,578 and 4,031,307 (the disclosures of which are incorporated herein by reference), such as guar gums comprising cationic trialkylammonium groups. Guar gums modified with a salt (e.g. chloride) of 2,3-epoxypropyltrimethylammonium can be used, for example.
   Such products are sold, for instance, under the trade names Jaguar C13 S, Jaguar C 15, Jaguar C 17, or Jaguar C162 by the company Meyhall.
(4) Polymers comprised of piperazinyl units and of divalent alkylene or hydroxyalkylene radicals comprising straight or branched chains, optionally interrupted by oxygen, sulphur, or nitrogen atoms or by aromatic or heterocyclic rings, and also the oxidation and/or quaternization products of these polymers. Such polymers are described, for example, in French patents 2 162 025 and 2 280 361 (the disclosures of which are incorporated herein by reference).
(5) Water-soluble polyamino amides prepared, for example, by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide, or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide, or a bis-unsaturated derivative; the crosslinking agent being used in proportions ranging from 0.025 to 0.35 mol per amine group of the polyamino amide; these polyamino amides can be alkylated or, if they contain one or more tertiary amine functions, they can be quaternized. Such polymers are described, for example, in French patents 2 252 840 and 2 368 508 (the disclosures of which are incorporated herein by reference).
(6) The polyamino amide derivatives resulting from the condensation of polyalkylene polyamines with polycarboxylic acids followed by alkylation with difunctional agents. Mention may be made, for example, of adipic
   acid/dialkylaminohydroxyalkyldialkylene-triamine polymers in which the alkyl radical comprises from 1 to 4 carbon atoms and preferably denotes methyl, ethyl, or propyl. Such polymers are described for example in French patent 1 583 363 (the disclosure of which is incorporated herein by reference).
   Among these derivatives, further mention may be made of the adipic acid/dimethylaminohydroxypropyl/diethylenetriamine polymers sold unde the name "Cartaretine F, F4 or F8" by the company Sandoz, for example.
(7) The polymers obtained by reaction of a polyalkylene polyamine comprising two primary amine groups and at least one secondary amine group with a dicarboxylic acid chosen from diglycolic acid and saturated aliphatic dicarboxylic acids comprising from 3 to 8 carbon atoms. The molar ratio between the polyalkylene polyamine and the dicarboxylic acid is between 0.8:1 and 1.4:1; the polyamino amide resulting therefrom is reacted with epichlorohydrin in a molar ratio of epichlorohydrin relative to the secondary amine group of the polyamino amide of between 0.5:1 and 1.8:1. Such polymers are described, for example, in U.S. Pat. Nos. 3,227,615 and 2,961,347 (the disclosures of which are incorporated herein by reference).
   Polymers of this type are sold under the name "Hercosett 57" by the company Hercules Inc. or alternatively under the name "PD 170" or "Delsette 101 " by the company Hercules, for example, in the case of the adipic acid/epoxypropyl/diethylenetriamine copolymer.
(8) Cyclopolymers of alkyldiallylamine or of dialkyldiallylammonium, such as the homopolymers or copolymers comprising, as the main constituent of the chain, units corresponding to formula (IX) or (X): 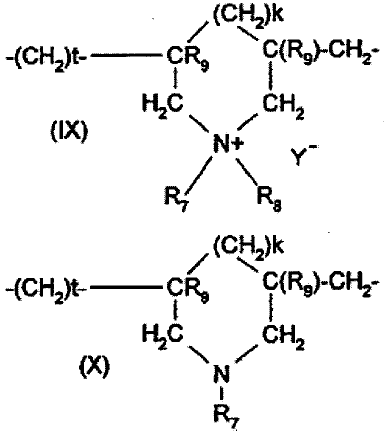 in which formulae k and t are equal to 0 or 1, the sum k+t being equal to 1; R₉ denotes a hydrogen atom or a methyl radical; R₇ and R₈, independently of each other, denote an alkyl group comprising from 1 to 6 carbon atoms, a hydroxyalkyl group in which the alkyl group comprises 1 to 5 carbon atoms, a lower C₁-C₄ amidoalkyl group, or R₇ and R₈ can denote, together with the nitrogen atom to which they are attached, heterocyclic groups such as piperidyl or morpholinyl; R₇ and R₈, independently of each other, denote an alkyl group comprising from 1 to 4 carbon atoms; Y⁻ is an anion such as bromide, chloride, acetate, borate, citrate, tartrate, bisulphate, bisulphite, sulphate, or phosphate. These polymers are described, for example, in French patent 2 080 759 and in its Certificate of Addition 2 190 406 (the disclosures of which are incorporated herein by reference).
   Among the polymers defined above, mention may be made, for example, of the dimethyldiallylammonium chloride homopolymer sold under the name "Merquat 100" by the company Calgon (and its homologues of low weight-average molecular mass) and copolymers of diallyldimethylammonium chloride and of acrylamide, sold under the name "Merquat 550".
(9) The quaternary diammonium polymer comprising repeating units corresponding to the formula: 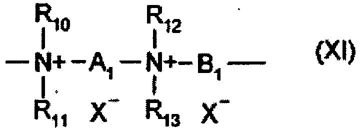 wherein:
   R_{10,} R₁₁, R₁₂ and R₁₃, which may be identical or different, represent aliphatic, alicyclic, or arylaliphatic radicals comprising from 1 to 6 carbon atoms or lower hydroxyalkylaliphatic radicals, or alternatively R₁₀, R₁, R₁₂ and R₁₃, together or separately, constitute, with the nitrogen atoms to which they are attached, heterocycles optionally comprising a second hetero atom other than nitrogen, or alternatively R₁₀, R_{11,} R₁₂ and R₁₃ represent a linear or branched C₁-C₆ alkyl radical substituted with a nitrile, ester, acyl, or amide group or a group -CO-O-R₁₄-D or -CO-NH-R₁₄-D where R₁₄ is an alkylene and D is a quaternary ammonium group;
   A₁ and B₁ represent polymethylene groups comprising from 2 to 6 carbon atoms which may be linear or branched, saturated or unsaturated, and which may comprise, linked to or intercalated in the main chain, one or more aromatic rings or one or more oxygen or sulphur atoms or sulphoxide, sulphone, disulphide, amino, alkylamino, hydroxyl, quaternary ammonium, ureido, amide, or ester groups, and
   X⁻ denotes an anion derived from a mineral or organic acid;
   A₁, R₁₀ and R₁₂ can form, with the two nitrogen atoms to which they are attached, a piperazine ring; in addition, if A₁ denotes a linear or branched, saturated or unsaturated alkylene or hydroxyalkylene radical, B₁ can also denote a group -(CH₂)ₙ-CO-DOC-(CH₂)ₙ- in which D denotes:
      a) a glycol residue of formula: -O-Z-O-, where Z denotes a linear or branched hydrocarbon-based radical or a group corresponding to one of the following formulae:

         - (CH₂-CH₂-O)ₓ-CH₂-CH₂-

         - [CH₂-CH(CH₃)-O_{y}-CH₂-CH(CH₃)-

         where x and y denote an integer from 1 to 4, representing a defined and unique degree of polymerization or any number from 1 to 4 representing an average degree of polymerization;
      b) a bis-secondary diamine residue such as a piperazine derivative;
      c) a bis-primary diamine residue of formula: -NH-Y-NH-, where Y denotes a linear or branched hydrocarbon-based radical, or alternatively the divalent radical

         -CH₂-CH₂-S-S-CH₂-CH₂-_{;}
      d) a ureylene group of formula: -NH-CO-NH-.

   Substituent X⁻ can be an anion such as chloride or bromide, for example.
   These polymers can have a number-average molecular mass of from 1,000 to 100,000.
   Polymers of this type are described, for example, in French patents 2 320 330, 2 270 846, 2 316 271, 2 336 434 and 2 413 907 and U.S. Pat. Nos. 2,273,780, 2,375,853, 2,388,614, 2,454,547, 3,206,462, 2,261,002, 2,271,378, 3,874,870, 4,001,432, 3,929,990, 3,966,904, 4,005,193, 4,025,617, 4,025,627, 4,025,653, 4,026,945 and 4,027,020 (the disclosures of which are incorporated herein by reference).
   It is also possible to use polymers which comprise repeating units corresponding to formula (XII) below: 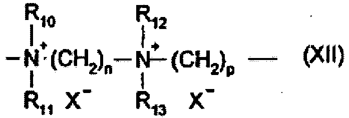 in which R₁₀, R₁₁, R₁₂ and R₁₃, which may be identical or different, denote an alkyl or hydroxyalkyl radical comprising from 1 to 4 carbon atoms approximately, n and p are integers ranging from 2 to 6 approximately, and X⁻ is an anion derived from a mineral or organic acid.
(10) Polyquaternary ammonium polymers comprised of repeating units of formula (XIII): 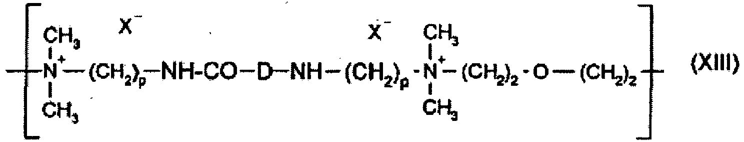 in which p denotes an integer ranging from 1 to 6 approximately, D may be nothing or may represent a group -(CH₂)ᵣ-CO- in which r denotes a number equal to 4 or 7, X⁻ is an anion.
   Such polymers may be prepared according to the processes described, for example, in U.S. Pat. Nos. 4,157,388, 4,702,906, and 4,719,282 (the disclosures of which are incorporated herein by reference). They are also described, for example, in patent application EP-A-122 324 (the disclosure of which is incorporated herein by reference).
   Among these products, mention may be made, for example, of "Mirapol A 15", "Mirapol AD1", "Mirapol AZ1" and "Mirapol 175" sold by the company Miranol.
(11) Quaternary polymers of vinylpyrrolidone and of vinylimidazole, such as, for example, the products sold under the names Luviquat FC 905, FC 550 and FC 370 by the company BASF.
(12) Polyamines such as Polyquart H sold by Henkel, which are given under the reference name "Polyethylene glycol (15) tallow polyamine" in the CTFA dictionary.

Other cationic polymers which can be used in the context of the invention are, polyalkyleneimines, polymers comprising vinylpyridine or vinylpyridinium units, condensates of polyamines and of epichlorohydrin, quaternary polyureylenes and chitin derivatives.

Other representative cationic polymers which may be used in the context of the present invention include polymers of (1), (9), (9), (10) and additional polymers comprised of repeating units of formulae (W) and (U) below: 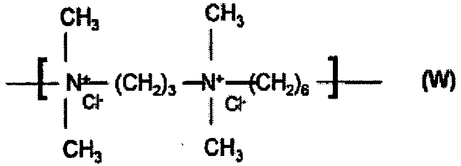 wherein the weight-average molar mass, determined by gel permeation chromatography, ranges from 9,500 to 9,900; 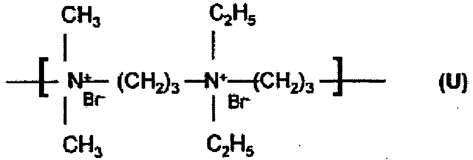 wherein the weight-average molar mass, determined by gel permeation chromatography, is about 1,200.

The amount of the cationic polymer may be from 0.0001 to 10 wt%, preferably 0.001 to 10 wt%, and more preferably 0.01 to 5 wt%, relative to the total weight of the composition.

The cosmetic composition according to the present invention may further comprise at least one higher alcohol. Two or more of the higher alcohols may be used.

The term "higher alcohol" here means any pure, saturated or unsaturated, linear or branched C₈-C₃₀ fatty alcohol.

Among the C₈-C₃₀ fatty alcohols, C₁₂-C₂₂ fatty alcohols are, for example, used. Mention may be made among these of lauryl alcohol, cetyl alcohol, stearyl alcohol, oleyl alcohol, behenyl alcohol, linoleyl alcohol, undecylenyl alcohol, palmitoleyl alcohol, linolenyl alcohol, arachidonyl alcohol and erucyl alcohol, and mixtures thereof. In one embodiment, cetyl alcohol, stearyl alcohol or a mixture thereof is used.

The amount of the higher alcohol may be 0.0001 to 10 wt%, preferably 0.001 to 10 wt%, and more preferably 0.01 to 5 wt%, relative to the total weight of the composition.

The cosmetic composition according to the present invention may also contain direct dyes for enriching the shades with glints. These direct dyes may then be chosen in particular from neutral, cationic or anionic nitro, azo or anthraquinone dyes in the proportion by weight of about 0.001 to 20 wt%, and preferably 0.01 to 10 wt% of the total weight of the composition.

The cosmetic composition according to the present invention may further comprise at least one fatty compound other than higher alcohols, as long as the purpose of the present invention is not hindered.

The fatty compound may be in the form of a liquid or a solid. Here, "liquid" means that the fatty compound is in the form of a liquid or a paste (non-solid) at room temperature (25°C) under atmospheric pressure (760 mmHg). As the liquid fatty compound, oils generally used in cosmetics can be used alone or in combination thereof.

The oil may be a non-polar oil such as a hydrocarbon oil, a silicone oil, or the like; a polar oil such as a vegetable oil and an ester oil; or a mixture thereof.

As examples of hydrocarbon oils, mention may be made of, for example, linear or branched hydrocarbons such as mineral oil (liquid paraffin), liquid vaseline, liquid naphthalene, and the like; hydrogenated polyisobutene, isoeicosan, squalane, squalene, and decene/butene copolymer; and mixtures thereof.

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as octamethylcyclotetrasiloxane, decamethylcyclopentasiloxane, dodecamethylcyclohexasiloxane, and the like; and mixtures thereof.

As examples of vegetable oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

The fatty compound may be a wax. Here, "wax" means that the fatty compound is substantially in the form of a solid at room temperature (25°C) under atmospheric pressure (760 mmHg), and has a melting point generally 35°C or more. As the waxy fatty substance, waxes generally used in cosmetics can be used alone or in combination thereof.

For example, the wax may be chosen from carnauba wax, microcrystalline waxes, ozokerites, hydrogenated jojoba oil, polyethylene waxes such as the wax sold under the name "Performalene 400 Polyethylene" by the company New Phase Technologies, silicone waxes, for instance poly(C₂₄-C₂₈)alkylmethyldimethylsiloxane, such as the product sold under the name "Abil Wax 9810" by the company Goldschmidt, palm butter, the C₂₀-C₄₀ alkyl stearate sold under the name "Kester Wax K82H" by the company Kester Keunen, stearyl benzoate, shellac wax, and mixtures thereof. For example, a wax chosen from carnauba wax, candelilla wax, ozokerites, hydrogenated jojoba oil and polyethylene waxes is used. In at least one embodiment, the wax is preferably chosen from candelilla wax and ozokerite, and mixtures thereof.

The cosmetic composition according to the present invention may comprise the fatty compound other than higher alcohols in an amount of 0.01 to 10 wt%, preferably 0.01 to 5 wt%, and more preferably 0.01 to 1 wt%, relative to the total weight of the composition.

The amount of the fatty compound may be 0.0001 to 10 wt%, preferably 0.001 to 10 wt%, and more preferably 0.01 to 5 wt%, relative to the total weight of the composition.

The cosmetic composition according to the present invention may also comprise an effective amount of other agents, known previously elsewhere in oxidation dyeing, such as various common adjuvants, for instance sequestering agents such as EDTA and etidronic acid, UV screening agents, other silicones than those mentioned before such as organomodified silicones (such as with amine groups), preserving agents, ceramides, pseudoceramides, vitamins or provitamins, for instance panthenol, opacifiers, etc.

The form of the cosmetic composition according to the present invention is not particularly limited, as long as it is water-based, and may take various forms such as an O/W emulsion, an aqueous gel, an aqueous solution, or the like.

The cosmetic composition according to the present invention is preferably a ready-to-use composition. For the purposes of the present invention, the expression "ready-to-use composition" is defined herein as a composition to be applied immediately to the keratin fibers. Said "ready-to-use composition" may be stored in unmodified form before use, or may result from the extemporaneous mixing of two or more separate compositions. One of the separate compositions may comprise one or more oxidation dyes, and another of the separate compositions may comprise one or more oxidizing agents. The amphoteric surfactant(s) is present in either or both of the separate compositions, the concentration of amphoteric surfactant(s) in the "ready-to-use composition" being 1.5 wt% or more relative to the total weight of the composition.

The above two or more separate compositions may be formulated into a multi-compartment system or kit in which a first compartment comprises one of the separate compositions and a second or subsequent compartment comprises another of the separate compositions. The multi-compartment system may be equipped with a means for mixing and/or applying the separate compositions such as a valve and a nozzle.

The pH of the cosmetic composition applied to the keratin fibers is generally, for example, from 4 to 12. It can range from 6 to 11 and may be adjusted to the desired value using acidifying or basifying agents that are well known in the prior art in the dyeing of keratin fibers.

Among the basifying agents which may be mentioned, for example, are aqueous ammonia, alkali metal carbonates, alkanolamines such as monoethanolamine, diethanolamine and triethanolamine and derivatives thereof, oxyethylenated and/or oxypropylenated hydroxyalkylamines and ethylenediamines, sodium hydroxide, potassium hydroxide and the compounds of formula (XXVI) below: 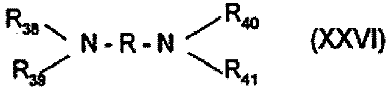 in which R is a propylene residue optionally substituted with a hydroxyl group or a C₁-C₄ alkyl radical; R₃₈, R₃₉, R₄₀ and R₄₁, which may be identical or different, represent a hydrogen atom, a C₁-C₄ alkyl radical, or a C₁-C₄ hydroxyalkyl radical.

The acidifying agents can be, for example, mineral or organic acids, for instance hydrochloric acid, orthophosphoric acid, carboxylic acids, for instance tartaric acid, citric acid, lactic acid, or sulphonic acids.

The cosmetic composition according to the present invention can be used in oxidation dyeing keratin fibers such as hair, comprising, for example, the steps of:
applying the ready-to-use cosmetic composition, prepared extemporaneously by mixing, just before oxidation dyeing the keratin fibers, two or more compositions described above, to wet or dry keratin fibers;
leaving the cosmetic composition to act for an exposure time, such as ranging from 1 to 60 minutes approximately, and further such as from 10 to 45 minutes approximately;
rinsing the fibers; and
optionally washing them with shampoo, rinsing them again and then drying them.

The application of the composition according to the present invention may be realized at room temperature or with the use of a warming device which is able to produce a temperature ranging from 40 to 220°C, preferably ranging from 40 to 80°C.

### EXAMPLES

The present invention will be described in more detail by way of examples, which however should not be construed as limiting the scope of the present invention.

### Example 1 and Comparative Examples 1 and 2

The following compositions according to Example 1 and Comparative Examples 1 and 2, shown in Table 1, were prepared by mixing the components shown in Table 1. Concentrations are expressed as commercial products.

**Table 1**

| Component | Ex. 1 | Comp. Ex. 1 | Comp Ex. 2 |
|---|---|---|---|
| Coco-Betaine (30%) | 6.00 | 1.00 | 1.00 |
| PEG-40 Hydrogenated Castor Oil | 0.32 | 0.32 | 0.32 |
| Sorbitol | 2.00 | - | - |
| Hexadimethrine Chloride (60%) | 0.04 | - | - |
| Polyquaternium-6 (40%) | 0.80 | - | - |
| Polyquaternium-22 | - | 0.40 | 0.40 |
| Fragrance | 0.16 | 0.16 | 0.16 |
| Erythorbic acid | 0.20 | - | - |
| Ascorbic acid | - | 0.08 | 0.08 |
| Sodium Metabisulfite | 0.20 | - | - |
| Sodium sulfite | - | 0.08 | 0.08 |
| Cysteine | - | 0.20 | 0.20 |
| p-Aminophenol | 0.07 | 0.24 | 0.07 |
| p-Phenylenediamine | 0.33 | 0.15 | 0.33 |
| Resorcinol | 0.34 | - | 0.34 |
| m-Aminophenol | 0.04 | - | 0.04 |
| 2-Methyl-5-hydroxyethylaminophenol | - | 0.32 | - |
| 4-Amino-2-hydroxytoluene | 0.04 | 0.36 | 0.04 |
| Ammonium Hydroxide (20% expressed in ammonia gas) | 1.90 | 4.00 | 4.00 |
| Ethanolamine | 2.0 | - | - |
| Cetearyl Alcohol | 0.15 | - | - |
| Sodium Laureth Sulfate (70%) | 0.3 | - | - |
| Glycerin | 2.4 | - | - |
| Polyquaternium-39 (9.25%) | 0.15 | - | - |
| Tetrasodium Etidronate | 0.12 | - | - |
| Tetrasodium Pyrophosphate | 0.024 | 0.012 | 0.012 |
| Sodium Salicylate | 0.021 | 0.021 | 0.021 |
| Hydrogen Peroxide (50%) | 7.02 | 7.20 | 7.20 |
| Water | qsp 100 | qsp 100 | qsp 100 |

### [Friction Evaluation]

Each formulation of Example 1 and Comparative Example 1 in an amount of 10 g was applied to 10 g of a lock of natural Japanese hair for 30 minutes at room temperature.

The frictional forces were measured on the locks of the natural dried hair using a sliding bench.

A mobile lock, attached to a sliding bench, is entrained in a horizontal rectilinear motion. The force to be exerted to make the lock slide is measured using an electronic gauge connected to the drive arm. The greater the sliding force, the greater the friction of the lock.

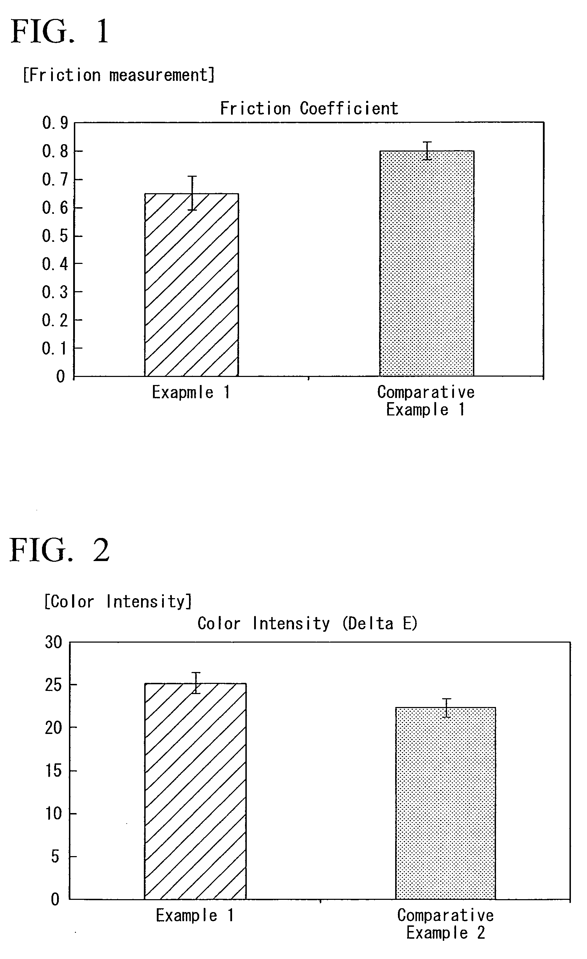

The results of the measurement are shown in Figure 1. As shown in Figure 1, the hair lock treated with Example 1 shows a lower friction coefficient than Comparative Example 1. These results demonstrate that Example 1 can provide a smoother feeling on touch.

### [Color Intensity Evaluation]

Each formulation of Example 1 and Comparative Example 2, which have the same dye components in the same amounts, in an amount of 1.0 g was applied to 1.0 g of a lock of white goat hair for 30 minutes at room temperature.

The color intensity (ΔE between dyed hair and non-dyed hair under L^{*}a^{*}b^{*} system) on the hair surface was determined by using a colorimeter (Konica-Minolta: CM-508d). For color intensity, 3 experiments were repeated and the result was calculated as the average of these 3 data.

The results of the measurement are shown in Figure 2. As shown in Figure 2, the hair lock treated with Example 1 showed higher color intensity than Comparative Example 2. These results demonstrate that Example 1 can provide the hair with better coloring effects.

## Claims

1. A cosmetic composition for oxidation dyeing keratin fibers comprising, in an aqueous medium:
(1) at least one oxidation dye;
(2) at least one oxidizing agent; and
(3) 1.5 wt% or more of at least one amphoteric surfactant relative to the total weight of the composition,
wherein the amphoteric surfactant is selected from C₈-C₂₀ alkylbetaines, and the composition further comprises at least one cationic polymer.

2. The composition according to claim 1, wherein the composition comprises water in an amount of 50 wt% or more relative to the total weight of the composition.

3. The composition according to claim 1 or 2, wherein the oxidation dye is selected from oxidation bases, oxidation couplers, and the acid addition salts thereof.

4. The composition according to claim 3, wherein the oxidation dye is an oxidation base selected from the group consisting of ortho- and para-phenylenediamines, double bases, ortho- and para-aminophenols, heterocyclic bases and the acid addition salts thereof.

5. The composition according to Claim 3, wherein the oxidation dye is an oxidation coupler selected from the group consisting of meta-phenylenediamines, meta-aminophenols, meta-diphenols, naphthols, heterocyclic couplers and the acid addition salts thereof.

6. The composition according to any one of Claims 1 to 5, wherein the amount of the oxidation dye(s) is 0.0001 to 20 wt%, preferably 0.0005 to 15 wt%, and more preferably 0.005 to 10 wt%, relative to the total weight of the composition.

7. The composition according to any one of Claims 1 to 6, wherein the oxidizing agent is selected from the group consisting of hydrogen peroxide, urea peroxide, alkali metal bromates, persalts, peracids and oxidase enzymes, and preferably is hydrogen peroxide.

8. The composition according to any one of Claims 1 to 7, wherein the amount of the oxidizing agent(s) is 0.0001 to 10 wt%, preferably 0.001 to 10 wt%, and more preferably 0.01 to 5 wt%, relative to the total weight of the composition.

9. The composition according to any one of Claims 1 to 8, wherein the amount of the amphoteric surfactant(s) is 1.5 to 10 wt%, preferably 1.8 to 5 wt%, and more preferably 1.8 to 3 wt% relative to the total weight of the composition.

10. The composition according to any one of Claims 1 to 9, wherein the composition further comprises at least one saturated or unsaturated, linear or branched, C₈₋₃₀ fatty alcohol.

11. The composition according to any one of Claims 1 to 10, wherein the composition is a mixture of a composition comprising one or several oxidation dyes and a composition comprising one or several oxidizing agents; wherein one or several amphoteric surfactants and cationic polymers are present in either or both of the separate compositions, and the concentration of amphoteric surfactant(s) in the composition is 1.5 wt% or more relative to the total weight of the composition.

## Patentansprüche

1. Kosmetische Zusammensetzung zur oxidativen Färbung von Keratinfasern, die in einem wässrigen Medium Folgendes umfasst:
(1) mindestens einen Oxidationsfarbstoff;
(2) mindestens ein Oxidationsmittel; und
(3) mindestens 1,5 Gew.-% mindestens eines amphoteren Tensids bezogen auf das Gesamtgewicht der Zusammensetzung,
wobei das amphotere Tensid aus C₈-C₂₀-Alkylbetainen ausgewählt ist und die Zusammensetzung ferner mindestens ein kationisches Polymer umfasst.

2. Zusammensetzung nach Anspruch 1, wobei die Zusammensetzung Wasser in einer Menge von mindestens 50 Gew.-% bezogen auf das Gesamtgewicht der Zusammensetzung umfasst.

3. Zusammensetzung nach Anspruch 1 oder 2, wobei der Oxidationsfarbstoff aus Oxidationsbasen, Oxidationskupplern und ihren Säureanlagerungssalzen ausgewählt ist.

4. Zusammensetzung nach Anspruch 3, wobei der Oxidationsfarbstoff eine Oxidationsbase ist, die aus der aus ortho- und para-Phenylendiaminen, Doppelbasen, ortho- und para-Aminophenolen, heterocyclischen Basen und ihren Säureanlagerungssalzen bestehenden Gruppe ausgewählt ist.

5. Zusammensetzung nach Anspruch 3, wobei der Oxidationsfarbstoff ein Oxidationskuppler ist, der aus der aus meta-Phenylendiaminen, meta-Aminophenolen, meta-Diphenolen, Naphtholen, heterocyclischen Kupplern und ihren Säureanlagerungssalzen bestehenden Gruppe ausgewählt ist.

6. Zusammensetzung nach einem der Ansprüche 1 bis 5, wobei die Menge des Oxidationsfarbstoffs (der Oxidationsfarbstoffe) 0,0001 bis 20 Gew.-%, vorzugsweise 0,0005 bis 15 Gew.-% und mehr bevorzugt 0,005 bis 10 Gew.-%, bezogen auf das Gesamtgewicht der Zusammensetzung, beträgt.

7. Zusammensetzung nach einem der Ansprüche 1 bis 6, wobei das Oxidationsmittel aus der aus Wasserstoffperoxid, Harnstoffperoxid, Alkalimetallbromaten, Persalzen, Persäuren und Oxidase-Enzymen bestehenden Gruppe ausgewählt ist und vorzugsweise Wasserstoffperoxid ist.

8. Zusammensetzung nach einem der Ansprüche 1 bis 7, wobei die Menge des Oxidationsmittels (der Oxidationsmittel) 0,0001 bis 10 Gew.-%, vorzugsweise 0,001 bis 10 Gew.-% und mehr bevorzugt 0,01 bis 5 Gew.-%, bezogen auf das Gesamtgewicht der Zusammensetzung, beträgt.

9. Zusammensetzung nach einem der Ansprüche 1 bis 8, wobei die Menge des amphoteren Tensids (der amphoteren Tenside) 1,5 bis 10 Gew.-%, vorzugsweise 1,8 bis 5 Gew.-% und mehr bevorzugt 1,8 bis 3 Gew.-, bezogen auf das Gesamtgewicht der Zusammensetzung, beträgt.

10. Zusammensetzung nach einem der Ansprüche 1 bis 9, wobei die Zusammensetzung ferner mindestens einen gesättigten oder ungesättigten, linearen oder verzweigten C₈₋₃₀-Fettalkohol umfasst.

11. Zusammensetzung nach einem der Ansprüche 1 bis 10, wobei die Zusammensetzung eine Mischung einer einen oder mehrere Oxidationsfarbstoffe umfassenden Zusammensetzung und einer ein oder mehrere Oxidationsmittel umfassenden Zusammensetzung ist; wobei ein oder mehrere amphotere Tenside und kationische Polymere in einer oder in beiden der getrennten Zusammensetzungen vorliegen und die Konzentration des amphoteren Tensids (der amphoteren Tenside) in der Zusammensetzung mindestens 1,5 Gew.-%, bezogen auf das Gesamtgewicht der Zusammensetzung, beträgt.

## Revendications

1. Composition cosmétique pour teinture d'oxydation de fibres kératiniques, comprenant, dans un milieu aqueux :
1) au moins un colorant d'oxydation,
2) au moins un agent oxydant,
3) et au moins 1,5 %, en poids rapporté au poids total de la composition, d'au moins un tensioactif amphotère,
étant entendu que le tensioactif amphotère est choisi parmi les (alkyle en C₈-C₂₀)-bétaïnes et que la composition comprend en outre au moins un polymère cationique.

2. Composition conforme à la revendication 1, laquelle composition comprend de l'eau en une proportion de 50 % ou plus, en poids rapporté au poids total de la composition.

3. Composition conforme à la revendication 1 ou 2, dans laquelle le colorant d'oxydation est choisi parmi les bases d'oxydation, les coupleurs d'oxydation, et leurs sels d'addition d'acide.

4. Composition conforme à la revendication 3, dans laquelle le colorant d'oxydation est une base d'oxydation choisie dans l'ensemble constitué par les ortho-phénylène-diamines, para-phénylène-diamines, bases doubles, ortho-amino-phénols, para-amino-phénols et bases hétérocycliques, ainsi que leurs sels d'addition d'acide.

5. Composition conforme à la revendication 3, dans laquelle le colorant d'oxydation est un coupleur d'oxydation choisi dans l'ensemble constitué par les méta-phénylène-diamines, méta-amino-phénols, méta-diphénols, naphtols et coupleurs hétérocycliques, ainsi que leurs sels d'addition d'acide.

6. Composition conforme à l'une des revendications 1 à 5, dans laquelle la proportion du ou des colorant(s) d'oxydation vaut de 0,0001 à 20 %, de préférence de 0,0005 à 15 %, et mieux encore de 0,005 à 10 %, en poids rapporté au poids total de la composition.

7. Composition conforme à l'une des revendications 1 à 6, dans laquelle l'agent oxydant est choisi dans l'ensemble constitué par les peroxyde d'hydrogène, peroxyde d'urée, bromates de métal alcalin, persels, peracides et enzymes de type oxydase, et de préférence, est du peroxyde d'hydrogène.

8. Composition conforme à l'une des revendications 1 à 7, dans laquelle la proportion du ou des agent(s) oxydant(s) vaut de 0,0001 à 10 %, de préférence de 0,001 à 10 %, et mieux encore de 0,01 à 5 %, en poids rapporté au poids total de la composition.

9. Composition conforme à l'une des revendications 1 à 8, dans laquelle la proportion du ou des tensioactif(s) amphotère(s) vaut de 1,5 à 10 %, de préférence de 1,8 à 5 %, et mieux encore de 1,8 à 3 %, en poids rapporté au poids total de la composition.

10. Composition conforme à l'une des revendications 1 à 9, laquelle composition comprend en outre au moins un alcool gras en C₈-C₃₀, linéaire ou ramifié, saturé ou insaturé.

11. Composition conforme à l'une des revendications 1 à 10, laquelle composition est un mélange d'une composition comprenant un ou plusieurs colorant(s) d'oxydation et d'une composition comprenant un ou plusieurs agent(s) oxydant(s), étant entendu qu'un ou plusieurs tensioactif(s) amphotère(s) et polymère(s) cationique(s) sont présents dans l'une ou l'autre des compositions séparées ou dans les deux, et que la concentration du ou des tensioactif(s) amphotère(s) dans la composition vaut 1,5 % ou plus, en poids rapporté au poids total de la composition.
